Europäisches Patentamt

European Patent Office

Office européen des brevets

(19)

(11) Veröffentlichungsnummer: **0 060 434**
**B1**

(12) **EUROPÄISCHE PATENTSCHRIFT**

(45) Veröffentlichungstag der Patentschrift: **12.11.86**

(21) Anmeldenummer: **82101566.6**

(22) Anmeldetag: **01.03.82**

(51) Int. Cl.⁴: **A 61 K 43/00, A 61 K 49/02**

(54) Verfahren zur Herstellung eines 131 Jod enthaltenden diagnostischen oder therapeutischen Mittels.

(30) Priorität: **13.03.81 DD 228270**

(43) Veröffentlichungstag der Anmeldung:
**22.09.82 Patentblatt 82/38**

(45) Bekanntmachung des Hinweises auf die
Patenterteilung:
**12.11.86 Patentblatt 86/46**

(84) Benannte Vertragsstaaten:
**AT DE**

(56) Entgegenhaltungen:
**DE-A-2 140 177**
**GB-A- 767 073**
**US-A-3 159 545**

**JOURNAL OF NUCLEAR MEDICINE,**
**PROCEEDINGS OF THE 28TH ANNUAL**
**MEETING, Band 22, Nr. 6, 16.-19. Juni 1981, Las**
**Vegas, Nev., USA, Seite P 74, T.A. HANEY et**
**al.: "A therapeutic and diagnostic I-131 capsule**
**formulation with minimal volatility and**
**maximal bioavailability"**

(73) Patentinhaber: **Akademie der Wissenschaften**
**der DDR**
**Rudower Chaussee 5**
**DDR-1199 Berlin (DD)**

(72) Erfinder: **Lincke, Hans-Joachim**
**Horkenweg 25**
**DDR-8122 Radebeul II (DD)**

(74) Vertreter: **Patentanwälte Beetz sen. - Beetz jun.**
**Timpe - Siegfried - Schmitt-Fumian**
**Steinsdorfstrasse 10**
**D-8000 München 22 (DE)**

## Beschreibung

Die Erfindung betrifft ein Verfahren zur Herstellung eines [131]J enthaltenden Mittels, welches insbesondere als oral verabreichbares Diagnostikum oder Therapeutikum bei pathologischen Prozessen der Schilddrüse verwendet werden kann.

Zur Diagnose und Therapie pathologischer Schilddrüsenerkrankungen werden üblicherweise mit einem [131]J enthaltenden Präparat gefüllte, oral verabreichbare Gelatinekapseln verwendet. Diese Kapseln werden hergestellt, indem ein üblicherweise aus Natriumhydrogenphosphat bestehendes inertes Sorbens vor oder nach dem Einbringen in die Kapsel mit einer [131]Jodid-Pufferlösung beladen und die Kapsel verschlossen wird.

Die bezüglich Handhabung und innerer bzw äußerer Kontaminationsgefahr sehr vorteilhaften Jodgelatinekapseln haben aber wesentliche andere Nachteile, die darin bestehen, daß insbesondere bei Kapseln mit hoher Aktivität (Therapiekapseln) ein Abdampfen von [131]J in so hohem Maße auftritt, daß es zum Beispiel in medizinischen Laboratorien ohne entsprechende lufttechnische Einrichtungen zu starken Überschreitungen der maximal zulässigen Konzentration von [131]J in der Raumluft kommen kann (Tomaru, T., et al, Radioisotopes, Tokyo, *28* (1979) S. 36—38). Außerdem nimmt die radiochemische Reinheit der Kapsel aufgrund von Jodmarkierungen an dem hauptsächlich organischen Material schnell ab, so daß nach Auflösung der Kapsel im Magensaft nur ein wesentlich verringerter Teil Jodid vorliegt, was zu einer verminderten Aufnahme von Jod in der Schilddrüse und damit zu einer Fehldiagnose bzw einem verringerten therapeutischen Effekt führen kann (Deckert, H. et al., Radiobiol. Radiotherapie *3* (1979) 279). Andererseits kann durch entstehende markierte Verbindungen eine unkontrollierte Jodbelastung in anderen Organen erfolgen.

Der Erfindung liegt die Aufgabe zugrunde, ein Verfahren zur Herstellung eines [131]J enthaltenden diagnostischen bzw therapeutischen Mittels mit geringer [131]J-Abdampfung und über längere Zeit stabil bleibender radiochemischer Reinheit anzugeben, bei dem durch geeignete Präparation des Sorbens das [131]J so gebunden wird, daß ein Abdampfen weitgehend verhindert und die Bildung undefinierter [131]J-Verbindungen unterbunden wird.

Erfindungsgemäß wird diese Aufgabe dadurch gelöst, daß dem inerten Sorbens vor der Jodsorption Ascorbinsäure und kristallwasserfreies Natriumthiosulfat zugemischt werden. Als inertes Sorbens wird üblicherweise Natriumhydrogenphosphat verwendet. Als günstiges Mischungsverhältnis hat sich dabei ein Verhältnis von 10 Teilen Inertmaterial zu je 1 Teil Inhibitor bzw Reduktionsmittel erwiesen. Durch den erfindungsgemäßen Zusatz der Ascorbinsäure werden die durch Radiolyse entstehenden, chemisch sehr aggressiven [131]J enthaltenden Radikale gebunden und damit die Bildung undefinierter, teilweise flüchtiger [131]J-Verbindungen verhindert. Durch das zugemischte Natriumthiosulfat werden die radiolytisch entstandenen bzw durch die Ascorbinsäure bereits gebundenen Radikale wieder in Jodid umgewandelt.

Nachfolgend wird die Erfindung anhand eines Ausführungsbeispiels näher erläutert.

### Beispiel

Für die Herstellung von 100 Therapiekapseln unterschiedlicher Aktivität werden 20 g $Na_2HPO_4 \cdot 2H_2O$, 2 g $Na_2S_2O_3$ und 2 g Ascorbinsäure miteinander vermischt. Die Mischung wird in Teilmengen von jeweils ca. 200 mg in Gelatinekapseln gefüllt und je nach der gewünschten Aktivität mit ca. 10—100 µl Jodid-Pufferlösung (zum Beispiel $Na^{131}J$ in $0,82 \cdot 10^{-2}$ M $Na_2CO_3$ und $1,1 \cdot 10^{-2}$ M $NaHCO_3$) je Kapsel aufgefüllt. Anschließend werden die Gelatinekapseln verschlossen und in entsprechenden Bleicontainern dem Anwender zugeführt. Mit den erfindungsgemäß hergestellten Kapseln konnte die Abdampfrate an [131]J-Aktivität um 1 bis 2 Größenordnungen, bezogen auf 200 1 Luft pro Stunde, gesenkt werden. Die erreichten Abdampfraten liegen somit bei $10^{-5}$ bis $10^{-6}$ % der Kapselaktivität. Der Jodidgehalt der Kapseln und damit die radiochemische Reinheit liegt selbst nach 20 Tagen noch bei Werten über 95 %.

## Patentansprüche

1. Verfahren zur Herstellung eines [131]J enthaltenden diagnostischen oder therapeutischen Mittels durch Sorption von radioaktivem [131]J in Form von Jodid an einem inerten Sorbens und Weiterverarbeitung zu oral verabreichbaren Kapseln, dadurch gekennzeichnet, daß dem inerten Sorbens vor der Sorption des [131]J Ascorbinsäure und kristallwasserfreies Natriumthiosulfat zugemischt werden.

2. Verfahren nach Anspruch 1, dadurch gekennzeichnet, daß die Ascorbinsäure sowie das Natriumthiosulfat jeweils in einer Menge von 1 Teil auf 10 Teile inertes Sorbens zugemischt werden.

## Revendications

1. Procédé de préparation d'une composition thérapeutique ou diagnostique contenant l'isotope 131 de l'iode par sorption de l'isotope radioactif 131 de l'iode sous forme d'iodure à un sorbant inerte et par transformation ultérieure en des capsules administrables oralement, caractérisé en ce que avant la sorption de l'isotope 131 de l'iode on mélange avec le sorbant de l'acide ascorbique et du thiosulfate de sodium sans eau de cristallisation.

2. Procédé selon la revendication 1, caractérisé en ce que l'acide ascorbique et le thiosulfate de sodium sont chacun mélangés avec le sorbant inerte dans une quantité d'une part sur 10 parts du sorbant inerte.

## Claims

1. Process for the preparation of a diagnostic or therapeutic composition containing iodine 131 by sorption of radioactive iodine 131 in form of iodide on an inert adsorbens and by subsequent processing in orally administrable capsules characterised in that before sorption of the iodine 131 ascorbic acid and sodiumthiosulfate without water of crystallization are admixed to the inert adsorbens.

2. Process according to Claim 1, characterised in that said ascorbic acid and said sodium thiusulfate each are admixed to the inert adsorbens with a relation of one part to 10 parts of said inert adsorbens.